# EUROPEAN PATENT APPLICATION

(11) **EP 4 582 111 A2**
(43) Date of publication of application: **09.07.2025**
(21) Application number: 25163744.3
(22) Date of filing: 05.03.2021
(51) Int. Cl.: A61L 27/60

(54) **A METHOD FOR PRODUCING A DECELLULARIZED TISSUE SCAFFOLD**

(30) Priority: 05.03.2020 GB 202003210
(62) Divisional of application: 21711331.5
(71) Applicant: Tissue Regenix Limited, Garforth, Leeds LS25 2GY (GB)
(72) Inventor: ROWLEY, Christine, Leeds, LS25 2GY (GB); STEVENSON, Grace, Leeds, LS25 2GY (GB); TAYLOR, Rebekah, Leeds, LS25 2GY (GB)
(74) Representative: HGF

(57) **Abstract**

The invention relates to a method of producing a decellularized tissue scaffold. The invention also relates to a tissue scaffold produced by said method. In particular, porcine tissue scaffolds. The method comprises reduced levels of anionic detergent, and avoids the use of animal derived protease inhibitors to produce a tissue scaffold with favourable properties.

## Description

### FIELD OF INVENTION

The present invention relates to a method of producing a decellularized tissue scaffold. The invention also relates to a tissue scaffold produced by said method.

### BACKGROUND

Soft tissue grafts of animal or human origin are used in a variety of medical procedures to surgically repair or replace soft tissue. For example, such grafts can be used to repair or reconstruct tissue in hernia repair, pelvic organ prolapse or ligament reconstruction.

In order for human tissue (allograft) or animal tissue (xenograft) to be used as a safe and effective tissue scaffold it is necessary to ensure that the graft is free from bacteria and viruses, is decellularized and is free from DNA to prevent graft rejection, and retains the functional properties of the native tissue it is derived from, including biomechanical properties and ability to function as an extracellular matrix (ECM).

It is known in the art that the use of anionic detergents such as sodium dodecyl sulphate (SDS) in addition to one or more protease inhibitors is highly effective in decellularising soft tissue whilst maintaining its ECM structure to create an effective biological scaffold (EP1392372B1). However, SDS even at relatively low concentrations is highly cytotoxic which may lead to failure in the biocompatibility of the graft due to residues of the detergent being retained in the graft. Additionally, certain protease inhibitors, such as aprotinin, are often of animal origin, which is associated with the risk of exposing the graft recipient to zoonotic diseases (for example transmissible spongiform encephalopathies including bovine spongiform encephalopathy), which is unacceptable in the context of medical products.

An additional problem with current processing methods is the effective removal of viruses from the grafts themselves, when non-human tissue is used. It is important to ensure that the xenograft is biologically safe by reducing potential zoonotic pathogens (e.g. porcine parvo virus (PPV) and porcine endogenous retrovirus (PERV)) to safe levels. For this reason, animal tissue must be exposed to a disinfection/sterilization process that has been proven to reduce zoonosis by up to 6-log. However, reduction of this magnitude is not typically achieved by traditional sterilisation methods e.g. gamma irradiation at a dose of 25 kGy.

The present invention aims to provide an improved method for producing a decellularized tissue scaffold which overcomes or partially ameliorates the problems associated with the methods known in the art.

### SUMMARY OF INVENTION

In one aspect, the present invention relates to a method for producing a decellularized tissue scaffold, the method comprising the steps of:
- incubating a starting tissue with a delipidating agent;
- incubating the tissue with an anionic detergent;
- incubating the tissue at least once with a hypotonic solution and at least once with a hypertonic solution, and/or subjecting the tissue to at least one freeze/thaw cycle; and
- incubating the tissue with a DNA removal agent, thereby obtaining a decellularized tissue scaffold.

It will be appreciated that the steps of the method of the invention do not need to be carried out in the order as provided above. Additionally, or alternatively, two or more of the steps may be combined into a single step. Merely by way of example, the step of incubating the tissue at least once with a hypotonic buffer and incubating the tissue with an anionic detergent may be combined into a single step. Exemplary sequences of the steps are provided elsewhere in the present specification.

Furthermore, multiple steps of each incubation may be comprised within the method. For example, the method may comprise multiple steps of incubating the tissue with a delipidating agent.

Suitably, the starting tissue may be selected from the group consisting of skin, meniscus, tendon, ligament, cartilage, muscle, blood vessel, and an organ.

Suitably, the delipidating agent may be a polar solvent. Suitably, the polar solvent may be acetone. Suitably, the delipidating agent may be at a concentration from about 80% to about 100% (v/v).

Suitably, the anionic detergent may be sodium dodecyl sulphate (SDS) or sodium deoxycholate. Suitably, the anionic detergent (for example SDS or sodium deoxycholate) may be at a concentration equal to or less than 0.2% (w/v).

Suitably, the method does not comprise a protease enzyme inhibitor. Suitably the method does not comprise an animal derived protease enzyme inhibitor. Suitably the method may comprise a non-animal derived protease inhibitor. Suitably the method may comprise a non-animal derived metalloprotease inhibitor. Suitably the non-animal derived protease inhibitor may be EDTA. Suitably, the non-animal derived protease enzyme inhibitor may be at a concentration of about 0.1% (w/v).

Suitably the method further comprises one or more rinse steps, suitably after each step of cell lysis, suitably therefore the one or more rinse steps take place after incubating the tissue at least once with a hypotonic solution and at least once with a hypertonic solution, and/or subjecting the tissue to at least one freeze/thaw cycle.

Suitably, the method does not comprise an alpha galactosidase enzyme.

Suitably, the DNA removal will be enabled by the use of a DNA removal agent which may be selected from the group consisting of an endonuclease enzyme or other treatments that break down DNA to enable elution from the tissue. The endonuclease enzyme may be selected from a human endonuclease selected from the group consisting of DNase Type I, DNase Type II, DNA Type III, and RNase, or other source including bacterially derived endonuclease, or any combination of two or more thereof. Suitably, the endonuclease enzyme may be a bacterially derived endonuclease, such as a genetically engineered Seratia marcescens endonuclease which may be selected from any commercially available endonuclease such as Benzonase ^{®}. Suitably, the endonuclease enzyme may be at a concentration from about 0.5 to about 50 U/ml. Suitably, the endonuclease enzyme is at a concentration of about 5 U/ml.

Suitably, the method may comprise the step of incubating the scaffold with an antimicrobial agent. Suitably, the antimicrobial agent may be an oxidizing agent. Suitably, the oxidizing agent may be peracetic acid. Suitably, the peracetic acid may be at a concentration from about 500 to about 10,000 ppm. Suitably at a concentration from about 0.05% to 1%.

Suitably, the method of the invention comprises the step of subjecting the tissue to ionizing radiation. Suitably, the ionizing radiation is at a dose of from about 15 to about 50 kGy.

Sutiably the method of the invention takes between 3 days to 14 days, suitably between 3 days to 12 days, suitably between 3 days to 10 days, suitably between 3 days to 7 days, suitably between 5 days to 14 days, suitably between 5 days to 12 days, suitably between 5 days to 10 days, suitably between 5 days to 7 days.

In a further aspect, the present invention relates to a decellularized tissue scaffold produced by a method of the invention.

Suitably, the decellularized tissue scaffold may be substantially free from anionic detergent (such as SDS) residues.

Suitably, the decellularized tissue scaffold may be substantially free from protease enzyme inhibitors, suitably free from animal-derived protease enzyme inhibitors.

Suitably, the decellularized tissue scaffold may be substantially free from galactose-alpha-1,3-galactose (also known as alpha-gal) protein.

Suitably, the tissue scaffold may be a complex tissue scaffold. Suitably, the complex tissue scaffold may be a meniscus tissue scaffold (for example porcine meniscus tissue scaffold), a dermis tissue scaffold (for example porcine dermis tissue scaffold), or a tendon tissue scaffold (for example porcine tendon tissue scaffold).

Except for where the context requires otherwise, the considerations set out in this disclosure should be considered to be applicable to the method and the scaffold in accordance with the invention.

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

Various aspects of the invention are described in further detail below.

### BRIEF DESCRIPTION OF THE DRAWINGS

Embodiments of the invention are further described hereinafter with reference to the accompanying drawings, in which:

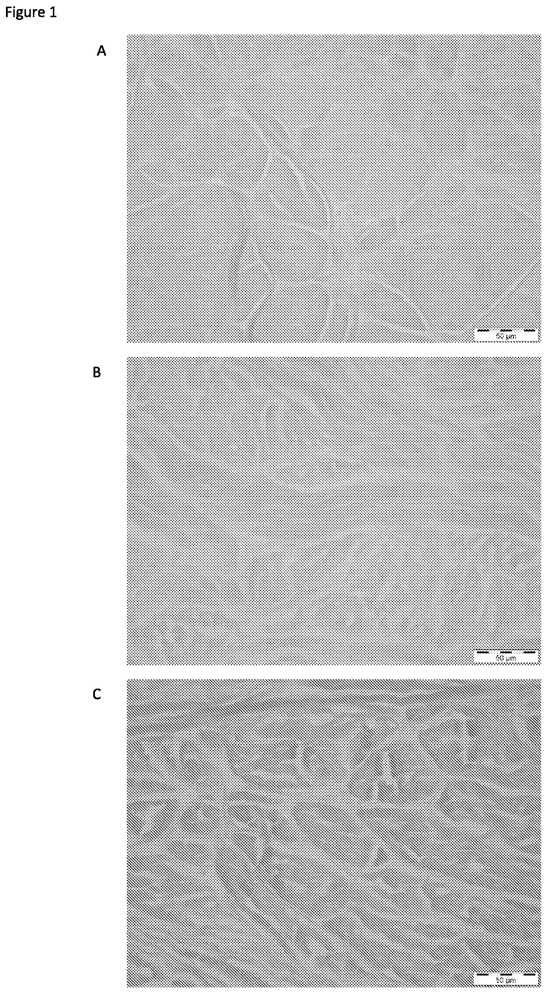

Figure 1 shows an image of a decellularized tissue scaffold produced by the method of the invention. In can be seen that the histoarchitecture of the material has been retained. A is a decellularized meniscus scaffold. B is a decellularized dermis scaffold. C is an alternative view of the decellularized dermis scaffold of figure 1B.

### DETAILED DESCRIPTION

The present invention is based on the inventors' development of a new method for preparing biological scaffolds with several advantages over prior methods.

Specifically, the inventors have found that by including at least one optimised step of incubation with a hypertonic solution and incubation with a hypotonic solution, and/or at least one optimised step of freezing/thawing the tissue, it is possible to significantly reduce the concentration of anionic detergent required to obtain a decellularized tissue scaffold.

Anionic detergents, such as SDS, are highly cytotoxic and leave processing residues in the tissue scaffold which can be cytotoxic and therefore negatively affect the scaffold's biocompatibility, even after SDS has been washed away. The inventors have found that it is possible to lower the concentration of anionic detergent (such as SDS) used, so that the tissue scaffold may be substantially free from processing residues, thereby improving biocompatibility of the scaffold, by modifying and optimising other steps in the decellularization process.

An additional advantage of the method of the invention is that it no longer requires the use of a protease enzyme inhibitor as is commonly used in the art. This is beneficial, as many methods use animal-derived protease inhibitors such as aprotinin which is of bovine origin and associated with a risk of zoonotic disease transmission. The inventors have found that the addition of rinse steps after cell lysis can sufficiently remove the proteases present in the decellularized tissue, and furthermore that reduction of processing time during each step of cell lysis can reduce the effects of proteases that are released from cells. This means that the use of a protease enzyme inhibitor is no longer required. Whilst reducing the risk of zoonotic disease transmission, this also increases the efficiency of the process.

Additionally, the method of the invention was also found to significantly reduce the amount of galactose-alpha-1,3-galactose (also known as alpha-gal) protein present in the decellularized tissue. This is advantageous as alpha-gal protein may cause an immune response in the subject. In the methods of the prior art, removal of alpha-gal generally required an additional step of treating the tissue with alpha galactosidase enzyme, which degrades the alpha-gal. The inventors have found that in the present method, this step is not necessary in order to remove substantially all alpha-gal from the scaffold. The method of the invention renders the scaffold substantially free from alpha-gal.

### Method Steps

The present invention relates to a method for producing a decellularized tissue scaffold, the method comprising the steps of:
- incubating a starting tissue with a delipidating agent;
- incubating the tissue with an anionic detergent;
- incubating the tissue at least once with a hypotonic buffer and at least once with a hypertonic buffer, and/or subjecting the tissue to at least one freeze/thaw cycle; and
- incubating the tissue with a DNA removal agent, thereby obtaining a decellularized tissue scaffold.

As mentioned elsewhere in the present specification, the steps of the method do not have to be carried out in the order provided above.

Suitably the method may in some cases comprise a step of incubating the tissue with a protease enzyme inhibitor. Suitably a non-animal derived protease inhibitor.

As such, the step of incubating the tissue with a delipidating agent may be carried out prior to the step of incubating the tissue with an anionic detergent, the step of incubating the tissue at least once with a hypotonic buffer and at least once with a hypertonic buffer, and/or subjecting the tissue to at least one freeze/thaw cycle, and the step of incubating the tissue with a protease enzyme inhibitor and incubating the tissue with a DNA removal agent.

Suitably, the step of incubating the tissue with an anionic agent may be carried out after the step of incubating the tissue with a delipidating agent.

Suitably, the step of incubating the tissue with an anionic agent may be carried out prior to the step of incubating the tissue with a DNA removal agent.

Suitably, the step of incubating the tissue with a DNA removal agent may be carried out after the step of incubation with an anionic agent.

Other variations in the order of the steps of the method will be known to those skilled in the art. Two or more of the steps of the invention may be combined into a single step.

Suitably, the step of incubating the tissue with an anionic detergent may be combined with the step of incubating the tissue with a hypotonic buffer.

Other ways in which two or more steps of the method of the invention may be combined, will be apparent to those skilled.

### Tissue Scaffold

The term "tissue scaffold" as used herein refers to a biological structural support designed to mimic the natural extracellular matrix. Suitably the tissue scaffold may facilitate attachment, migration, proliferation, and/or three-dimensional organisation of cells growing therein.

The decellularized tissue scaffold may be produced by the method of the invention from tissue obtained from a human or animal subject, or human or animal cadaver. In the context of the present disclosure, the tissue obtained from a human or animal subject used to make the decellularized tissue scaffold may be referred to as "a starting tissue" or "a tissue".

Suitably, the starting tissue may be selected from the group consisting of skin (or part of skin such as dermis), meniscus, tendon, ligament, cartilage, muscle, blood vessel, and an organ (such as a heart, kidney, liver, etc.). Suitably, the starting tissue may be skin or meniscus or tendon. Merely by way of example, the starting tissue may be dermis, such as porcine dermis, or the starting tissue may be porcine meniscus or porcine tendon.

Suitably, the animal from which the starting tissue is obtained, may be for example, a pig, a non-human primate, a cow, a sheep, or a horse.

In the context of present disclosure, the term "decellularized" means that the scaffold is substantially free from cellular components (such as cellular membranes, nucleic acids, lipids, lipid-like substances and cytoplasmic components). However, it will be appreciated that a decellularized scaffold will retain an extracellular matrix (ECM).

The phrase "extracellular matrix (ECM)" as used herein, refers to a network of materials produced and secreted by the cells of the tissue into the surrounding extracellular space and/or medium and which typically together with the cells of the tissue impart the tissue its mechanical and structural properties. Generally, the ECM includes fibrous elements (particularly collagen, elastin, or reticulin), cell adhesion polypeptides (such as fibronectin, laminin and adhesive glycoproteins), and space-filling molecules (such as glycosaminoglycans (GAGs), proteoglycans).

By "substantially free from" cellular material, it is meant that the scaffold is substantially free from cellular components and/or substantially free from nucleic acids.

The scaffold may be determined as substantially free from cellular components by microscopy using haematoxylin and eosin staining. Suitably the scaffold is at least 80% devoid of cellular components which naturally occur in the starting tissue from which the scaffold is produced. Suitably, the scaffold may be at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.5%, at least 99.9% or more devoid of cellular components.

The scaffold may be determined as substantially free from cellular material by measuring the remaining nucleic acid quantity in the scaffold after processing. Suitably the scaffold is also substantially free from nucleic acids, as defined hereinbelow. Suitably, the DNA may be sufficiently removed from the scaffold if the DNA amount is less than about 50 ng/mg. Suitably, if the DNA is less than about 50ng/mg, the scaffold is substantially free of cellular material. Suitably the DNA amount is less than about 50ng/mg dry weight of the scaffold.

Suitably therefore, the scaffold is substantially free from cellular material when either: (i) it is at least 80% devoid of cellular components, as defined above, or (ii) the DNA content is less than about 50ng/mg.

Suitably the scaffold is substantially free from cellular material when: (i) it is at least 80% devoid of cellular components, as defined above, and (ii) the DNA content is less than about 50ng/mg.

The extent of decellularization can be determined histochemically, for example, by staining the tissue with haematoxylin and eosin using standard techniques which visualizes cell membranes and nuclei. Immunohistochemical staining is another way in which the extent of decellularization can be determined. Immunohistochemical staining may visualize cell specific markers such as smooth muscle actin and histocompatibility antigens - an absence of such markers being an indication of decellularization. Another method in which the extent of decellularization can be determined is DNA quantification. DNA quantity of equal to or less than 50n/mg may be used as an indicator of decellularization. Other methods suitable for assessing decellularization will be known to those skilled in the art.

Suitably, the decellularized tissue scaffold may be substantially free from alpha-gal. In this context, "substantially free" means that the amount of alpha-gal in the scaffold is within clinically acceptable levels. Suitably, the scaffold may comprise 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, 1% or less of alpha-gal as compared to the amount of alpha-gal naturally occurring in the starting tissue from which the scaffold is produced. Methods for determining the amount of alpha-gal will be known to those skilled in the art. Merely by way of example, alpha-gal may be measured by performing an ELISA using commercially available kits, such as that available from MyBioSource.

### Delipidating Agent

In the method of the invention, the starting tissue is incubated with a delipidating agent. The term "delipidating agent" as used herein refers to an agent that removes lipids and/or lipid-like substance from the starting tissue such that the starting tissue is substantially free from lipids and/or lipid-like substances. The lipids and/or lipid-like substances may include, but are not limited to, complex lipids, simple lipids, triglycerides, fatty acids, glycerophospholipids (phospholipids), true fats such as esters of fatty acids, glycerol, cerebrosides, waxes, and sterols such as cholesterol and ergosterol. The lipids and/or lipid-like substances may be derived from the starting tissue itself.

In the context of a delipidating agent, by "substantially free from", it is meant that the tissue is at least 80% devoid of lipids and/or lipid-like substances present on the tissue prior to incubation with the delipidating agent. Suitably, the tissue may be at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.5%, at least 99.9% or more devoid of lipids and/or lipid-like substances compared to the starting tissue.

The lipids and/or lipid-like substances may be insoluble in water and therefore affect subsequent water-based treatments such as washes. Suitably, the lipids and/or lipid-like substances may be soluble in a polar solvent. Accordingly, the delipidating agent may be a polar solvent. The term "polar solvent" as used herein refers to a solvent that interacts with other compounds through acid-base interactions, hydrogen bonding, dipole- dipole interactions, and/or by dipole-induced dipole interactions.

Suitably, the polar solvent may be selected from the group consisting of acetone, isopropyl alcohol, methanol, ethanol, ethyl acetate, or a mixture of two or more thereof. More suitably, the polar solvent may be acetone.

Suitably, the polar solvent may be at a concentration from about 80% to about 100% v/v. In one embodiment, the polar solvent is at a concentration of about 99% v/v.

It will be appreciated that in the method of the present invention, the tissue may be incubated with a delipidating agent for a sufficient period of time to remove lipids and/or lipid-like substances from the tissue. Suitably, until the desired lipids and/or lipid-like substances are removed leaving the tissue substantially free from said lipids and/or lipid-like substances.

Suitably, the incubation period with a delipidating agent may be from about 10 minutes to about 24 hours, from about 1 hour to about 5 hours, suitably from about 2 hours to about 3 hours.

Suitably, the incubation period with a delipidating agent may be about 10 minutes, about 20 minutes, about 30 minutes, about 40 minutes, about 50 minutes, about 1 hour, about 2 hours, about 3 hours, about 4 hours, about 5 hours, about 10 hours, about 15 hours, about 20 hours, about 24 hours, or more. Suitably, the incubation period may be about 2 to 3 hours.

In one embodiment, the delipidating agent is acetone at a concentration of about 99%v/v. In such an embodiment the incubation period is about 2-3 hours.

In one embodiment, the incubation period is about 2 hours.

In one embodiment, the incubation period is about 3 hours.

In one embodiment, there may be more than one step of incubating the tissue with a delipidating agent. In one embodiment, there may be more than one incubation period with a delipidating agent. In some embodiments there may be two periods of incubation with a delipidating agent. Suitably each period of incubation may be a different total length of time and may comprise different blocks of time.

In one embodiment, the method comprises the steps of:
- incubating a starting tissue with a delipidating agent for about 2-3 hours, wherein the delipidating agent is 99% (v/v) acetone;
- incubating the tissue with an anionic detergent;
- incubating the tissue at least once with a hypotonic buffer and at least once with a hypertonic buffer, and/or subjecting the tissue to at least one freeze/thaw cycle; and
- incubating the tissue with a DNA removal agent, thereby obtaining a decellularized tissue scaffold.

The term "incubation" or "incubating" as used herein refers to contacting the tissue with a relevant agent (the relevant agent being a delipidating agent, anionic detergent, hypotonic solution, hypertonic solution, non-animal derived protease enzyme inhibitor, DNA removal agent, or antimicrobial agent). In the context of the present disclosure, the incubation period refers to the total amount of time that the tissue is contacted with (i.e. incubated with) the relevant agent.

The incubation period may be one continuous block of time equal to the total incubation period, or a plurality of blocks of time that combined equal the total incubation period. Merely by way of example, in the context of incubation with acetone, the incubation period may be comprised of one continuous block of time, or for example, three blocks of time.

It will be appreciated by the skilled person that between blocks of time there may be a period of rest. Suitably, the tissue may rest between the blocks of time. Suitably the relevant solution may be replaced between one or more blocks of time, for example so that the tissue is incubated with a fresh relevant solution. For example, the incubation period with a delipidating agent may be 2 hours comprised of three 40 minute blocks of time, wherein between each block of time the delipidating agent is replaced.

In one embodiment, the incubation period with a delipidating agent is 2 hours comprised of three 40 minute blocks of time.

In one embodiment, the incubation period with a delipidating agent is 3 hours comprised of three 1 hour blocks of time.

In one embodiment, the incubation period with a delipidating agent is 33 hours comprised of two 1 hour blocks of time and an extended block of 31 hours of time.

In one embodiment, the method comprises the steps of:
- incubating a starting tissue with a delipidating agent for about 3 hours, wherein the delipidating agent is 99% (v/v) acetone, and wherein the incubation is comprised of three 1 hour blocks of time;
- incubating the tissue with an anionic detergent;
- incubating the tissue at least once with a hypotonic buffer and at least once with a hypertonic buffer, and/or subjecting the tissue to at least one freeze/thaw cycle; and
- incubating the tissue with a DNA removal agent, thereby obtaining a decellularized tissue scaffold.

In one embodiment, the method comprises the steps of:
- incubating a starting tissue with a delipidating agent for about 2 hours, wherein the delipidating agent is 99% (v/v) acetone, and wherein the incubation is comprised of three 40 minute blocks of time;
- incubating the tissue with an anionic detergent;
- incubating the tissue at least once with a hypotonic buffer and at least once with a hypertonic buffer, and/or subjecting the tissue to at least one freeze/thaw cycle; and
- incubating the tissue with a DNA removal agent, thereby obtaining a decellularized tissue scaffold.

In one embodiment, the method comprises only one step of incubating a starting tissue with a delipidating agent. In one embodiment, the step is for about 2 hours, wherein the delipidating agent is 99% (v/v) acetone, and wherein the incubation is comprised of three 40 minute blocks of time. In another embodiment, the method comprises two steps of incubating a starting tissue with a delipidating agent. In one embodiment, the first step may be for about 33 hours and the second step for about 3 hours, wherein the first step comprises two 1 hour blocks of time and one 31 hour block of time, and wherein the second step comprises three 1 hour blocks of time.

### Anionic Detergent

The method of the invention comprises the step of incubating the tissue in an anionic detergent.

Suitably, incubation with an anionic detergent may be performed before or after incubation with a delipidating agent. More suitably, this step may be performed after incubation with a delipidating agent.

The term "anionic detergent" as used herein refers to a surfactant that has a negatively charged hydrophilic end. The anionic detergent may disrupt membranes and denature proteins by breaking protein-protein interactions.

Suitably, the anionic detergent may be selected from the group consisting of sodium dodecyl sulfate (SDS), sodium deoxycholate, sodium laurate, sodium stearate, dioctyl sodium sulfosuccinate, amphoteric sodium N-lauroyl sarcosinate, and mixture of any two or more thereof. More suitably, the anionic detergent is selected from the group consisting of: SDS, sodium deoxycholate, alkylbenzene sulfonates, alkyl sulfonates, alkyl sulfonates, alkyl sulfates, salts of fluorinated fatty acids, silicones, fatty alcohol sulfates, polyoxyethylene fatty alcohol ether sulfates, α-olefin sulfonate, polyoxyethylene fatty alcohol phosphates ether, alkyl alcohol amide, alkyl sulfonic acid acetamide, alkyl succinate sulfonate salts, amino alcohol alkylbenzene sulfonates, naphthenates, alkylphenol sulfonate and polyoxyethylene monolaurate In one embodiment, the anionic detergent is SDS.

Suitably, the anionic detergent (for example SDS or sodium deoxycholate) may be at a concentration from about 0.001% to about 0.1% (w/v), from about 0.002% to about 0.018% (w/v), from about 0.004% to about 0.016% (w/v), from about 0.006% to about 0.014% (w/v), or from about 0.008 to about 0.012% (w/v).

Suitably, the anionic detergent (for example SDS or sodium deoxycholate) may be at a concentration of about 0.2% or less (w/v), about 0.18% or less (w/v), about 0.16% or less (w/v), about 0.14% or less (w/v), about 0.12% or less (w/v), about 0.1% or less (w/v), about 0.08% or less (w/v), about 0.06% or less (w/v), about 0.04% or less (w/v), about 0.02% or less (w/v), about 0.01% or less (w/v), about 0.008% or less (w/v), about 0.006% or less (w/v), about 0.004% or less (w/v), about 0.002% or less (w/v), or about 0.001% or less (w/v).

In one embodiment, the anionic detergent (for example SDS or sodium deoxycholate) is at a concentration of about 0.01% (w/v).

In one embodiment, the anionic detergent (for example SDS or sodium deoxycholate) is at a concentration of about 0.1% (w/v).

The anionic detergent may be diluted to achieve the desired concentration in any suitable solution. The solution may be hypotonic, isotonic, or hypertonic solution. More suitably, the solution may be hypotonic.

Suitably, the solution may be selected from the group consisting of tris buffer, tris/EDTA buffer (0.1% tris/0.1% EDTA), tris[hydroxylmethyl]-aminomethane hydrochloride (Tris-HCl, 10-100 mM), (4-(2- hydroxyethyl)-I-piperazineethanesulfonic acid (HEPES, 10-100 mM), a dilute solution of PBS (such as a solution containing 0.2 grams KC1, 0.2 grams KH₂P0₄, 8 grams NaCl, or a solution containing 2.16 grams Na₂HP0₄*7H₂0 in 1000 ml H₂O, and a dilute solution of normal saline (for example containing 0.9% NaCl).

In one embodiment, the solution is hypotonic tris buffer.

Suitably, the incubation period with the anionic detergent (for example SDS or sodium deoxycholate) is for a sufficient time to achieve cell lysis in the tissue. Suitably, this may be from about 10 to about 100 hours, suitably from about 20 to about 80 hours.

Suitably, the incubation period with the anionic detergent may be about 10 hours, about 12 hours, about 14 hours, about 16 hours, about 18 hours, about 20 hours, about 22 hours, about 24 hours, about 26 hours, about 28 hours, about 30 hours, about 35 hours, about 40 hours, about 45 hours, about 50 hours, about 55 hours, about 60 hours, about 65 hours, about 70 hours, about 75 hours, about 80 hours, about 85 hours, about 90 hours, about 95 hours, about 100 hours, or more.

It will be appreciated that the incubation period with an anionic detergent may depend upon the type of the starting tissue used and/or the detergent itself. Merely by way of example, porcine dermis may be incubated with an anionic detergent (such as SDS) for about 21 or 24 hours, whereas porcine meniscus may be incubated with an anionic detergent (such as SDS)for about 80 hours, whereas porcine tendon may be incubated with an anionic detergent for about 48 hours. The skilled person is able to determine the optimum length of incubation with an anionic detergent, suitably by determining if cell lysis has occurred in the tissue, this may be determined using microscopy, for example.

In one embodiment, the method comprises the steps of:
- incubating a starting tissue with a delipidating agent for about 2-3 hours, wherein the delipidating agent is 99% (v/v) acetone;
- incubating the tissue with an anionic detergent, wherein the anionic detergent is 0.01% (w/v) SDS;
- incubating the tissue at least once with a hypotonic buffer and at least once with a hypertonic buffer, and/or subjecting the tissue to at least one freeze/thaw cycle; and
- incubating the tissue with a DNA removal agent, thereby obtaining a decellularized tissue scaffold.

In one embodiment, the tissue is incubated with anionic detergent 0.01% SDS for 21 or 24 hours.

In one embodiment, the tissue is incubated with anionic detergent 0.01% SDS for 80 hours comprised of four 20 hour blocks of time.

In one embodiment, the method comprises two steps of incubating the tissue with anionic detergent 0. 1% SDS. In one embodiment, each step is about 24 hours.

In one embodiment, the method comprises the steps of:
- incubating a starting tissue with a delipidating agent for about 3 hours, wherein the delipidating agent is 99% (v/v) acetone, and wherein the incubation is comprised of three 1 hour blocks of time;
- incubating the tissue with an anionic detergent for about 24 hours, wherein the anionic detergent is 0.01% (w/v) SDS;
- incubating the tissue at least once with a hypotonic buffer and at least once with a hypertonic buffer, and/or subjecting the tissue to at least one freeze/thaw cycle; and
- incubating the tissue with a DNA removal agent, thereby obtaining a decellularized tissue scaffold.

In one embodiment, the method comprises the steps of:
- incubating a starting tissue with a delipidating agent for about 2 hours, wherein the delipidating agent is 99% (v/v) acetone, and wherein the incubation is comprised of three 40 minute blocks of time;
- incubating the tissue with an anionic detergent for about 80 hours, wherein the anionic detergent is 0.01% (w/v) SDS, and wherein the incubation is comprised of four 20 hour blocks of time;
- incubating the tissue at least once with a hypotonic buffer and at least once with a hypertonic buffer, and/or subjecting the tissue to at least one freeze/thaw cycle; and
- incubating the tissue with a DNA removal agent, thereby obtaining a decellularized tissue scaffold.

### Hypotonic and Hypertonic Solutions

The method of the invention incudes the step of incubating the tissue at least once with a hypotonic solution and at least once with a hypertonic solution, and/or subjecting the tissue to at least one freeze/thaw cycle. Suitably these steps act to lyse the cells present in the tissue.

Suitably, incubating the tissue at least once with a hypotonic solution and at least once with a hypertonic solution achieves a similar effect as subjecting the tissue to at least one freeze/thaw cycle; both steps act to lyse cells. Suitably either step may be used, or both in combination. Sutiably, therefore the method may comprise incubating the tissue at least once with a hypotonic solution and at least once with a hypertonic solution, or suitably the method may comprise subjecting the tissue to at least one freeze/thaw cycle. Alternatively, the method may comprise incubating the tissue at least once with a hypotonic solution and at least once with a hypertonic solution, and subjecting the tissue to at least one freeze/thaw cycle.

By "freeze/thaw cycle" it is meant that the tissue is frozen (for example by being placed in liquid nitrogen, or into a -80°C or -20°C freezer) followed by being thawed (for example at 2-8°C or 34°C or 37°C or at room temperature).

Suitably the tissue may be subjected to multiple freeze/thaw cycles. Suitably the tissue may be subjected to between one to five freeze/thaw cycles. In one embodiment, the tissue is subjected to two or three freeze/thaw cycles.

The term "hypotonic solution" refers to a solution that has a concentration of solutes that is less than the concentration of solutes within a tissue cell. The term "hypertonic solution" refers to a solution that has a concentration of solutes that is more than the concentration of solutes within a tissue cell.

Merely by way of example, a hypotonic solution may be selected from the group consisting of a tris/EDTA buffer (0.1% tris/0.1% EDTA), tris[hydroxylmethyl]- aminomethane hydrochloride (Tris-HCl, 10-100 mM), (4-(2- hydroxyethyl)-I-piperazineethanesulfonic acid (HEPES, 10-100 mM), a dilute solution of PBS (such as a solution containing 0.2 grams KC1, 0.2 grams KH₂PO₄, 8 grams NaCl, or a solution containing 2.16 grams Na₂HPO₄*7H₂O in 1000 ml H₂O, and a dilute solution of normal saline (for example containing 0.9% NaCl). Other hypotonic solutions will be well known to those skilled in the art.

In one embodiment, the hypotonic solution is 0.1% tris/0.1% EDTA.

The incubation period with a hypotonic solution may be from about 30 minutes to 100 hours, suitably 10 hours to about 100 hours, or from about 20 hours to 80 hours.

Suitably, the incubation period with a hypotonic solution may be about 30 minutes, 1 hour, 5 hours, 10 hours, about 12 hours, about 14 hours, about 16 hours, about 18 hours, about 20 hours, about 22 hours, about 24 hours, about 26 hours, about 28 hours, about 30 hours, about 35 hours, about 40 hours, about 45 hours, about 50 hours, about 55 hours, about 60 hours, about 65 hours, about 70 hours, about 75 hours, about 80 hours, about 85 hours, about 90 hours, about 95 hours, about 100 hours, or more.

It will be appreciated that the incubation period with a hypotonic solution may depend upon the type of starting tissue used and/or the hypotonic solution itself. For example, a solution that is more hypotonic may require a shorter incubation period than a solution that is less hypotonic. Merely by way of example, porcine dermis may be incubated in a hypotonic solution for about 21-24 hours, whereas porcine meniscus may be incubated with a hypotonic solution for about 40 minutes, whereas porcine tendon may be incubated with a hypotonic solution for up to 24 hours, suitably in some cases for 1-4 hours, and in some cases for 24 hours. A suitable incubation time may be determined by the skilled person, by assessing when the tissue is decellularized using the methods described above, for example.

As touched upon elsewhere in the present specification, the anionic detergent may be diluted to the desired concentration in a hypotonic solution. In such an embodiment the step of incubating the tissue with an anionic detergent and the step of incubating the tissue at least once with a hypotonic solution may be combined into a single step.

In one embodiment, therefore, the method comprises the steps of:
- incubating a starting tissue with a delipidating agent;
- incubating the tissue with an anionic detergent and a hypotonic solution;
- incubating the tissue at least once with a hypertonic buffer, and/or subjecting the tissue to at least one freeze/thaw cycle; and
- incubating the tissue with a DNA removal agent, thereby obtaining a decellularized tissue scaffold.

The method of the invention comprises the step of at least once incubating the tissue with a hypotonic solution. Suitably, the tissue may be incubated one time, two times, three times or more with a hypotonic solution. In one embodiment, the tissue may be incubated two times with a hypotonic solution. In one embodiment, the tissue may be incubated six times with a hypotonic solution

Suitably, when the tissue is incubated two or multiple times with a hypotonic solution, one of the incubations may be combined with the step of incubating the tissue with an anionic detergent. This can be achieved by diluting the anionic detergent to a desired concentration in a hypotonic agent. Suitably, the second incubation with a hypotonic solution can be performed before or after the incubation with an anionic detergent.

In one embodiment, therefore, the method comprises the steps of:
- incubating a starting tissue with a delipidating agent;
- incubating the tissue with an anionic detergent and a first hypotonic solution;
- incubating the tissue at least once with a second hypotonic solution and at least one with a hypertonic solution, and/or subjecting the tissue to at least one freeze/thaw cycle; and
- incubating the tissue with a DNA removal agent, thereby obtaining a decellularized tissue scaffold.

Suitably, where the incubation with a hypotonic solution is performed before the step of incubating with an anionic detergent, it may be performed before or after the step of incubating with a delipidating agent. More suitably, where the incubation with a hypotonic solution is performed before the step of incubating with an anionic detergent, it may be performed after the step of incubating with a delipidating agent. The step of incubating the tissue in a hypotonic solution may be performed before or after subjecting the tissue to at least one freeze/thaw cycle. Suitably, the step of incubating the tissue in a hypotonic solution may be performed before subjecting the tissue to at least one freeze/thaw cycle. The step of incubating the tissue with a hypotonic solution may be followed by the step of incubating the tissue with a hypertonic solution. The incubation with a hypertonic solution may be directly after incubation with a hypotonic solution. Alternatively, the incubation with a hypertonic solution may be after the tissue has been subjected to at least one freeze/thaw cycle.

Suitably, in an embodiment where the incubation with a hypotonic solution is performed before the step of incubating with an anionic detergent, the method may comprise the steps of:
- incubating a starting tissue with a delipidating agent;
- incubating the tissue with a first hypotonic solution;
- subjecting the tissue to at least one freeze/thaw cycle;
- incubating the tissue with a first hypertonic solution;
- incubating the tissue with an anionic detergent and optionally a second hypotonic solution;
- incubating the tissue with a second hypertonic solution; and
- incubating the tissue with a DNA removal agent, thereby obtaining a decellularized tissue scaffold.

Suitably, where the incubation with a hypotonic solution is performed after incubation with an anionic detergent, it may also be performed after incubation of the tissue with a delipidating agent and/or incubation with a hypertonic solution. In such an embodiment, the method may comprise the steps of:
- incubating a starting tissue with a delipidating agent;
- incubating the tissue with an anionic detergent;
- incubating the tissue with a hypertonic solution;
- incubating the tissue with a hypotonic solution; and
- incubating the tissue with a DNA removal agent, thereby obtaining a decellularized tissue scaffold.

Suitably the first and second solutions may be the same solution or different solutions. Suitably the first and second solutions of any of the components of the process are selected from the components described herein. The same applies to any number of solutions of the same component, for example, third and fourth hypertonic solutions may be used, suitably these may all be the same solution or different solutions selected from those described herein.

In one embodiment the method may comprise six steps of incubating the tissue with hypotonic treatment. Suitably the first two steps comprise incubation with a hypotonic solution for a period of 1-4 hours. Suitably the third, fourth, fifth and sixth steps comprise incubation with a hypotonic solution for a period of 24 hours. Suitably at least one of the third, fourth, fifth and sixth steps comprises an anionic detergent. Suitably the first and second steps may each be prior to a freeze/thaw cycle.

In one embodiment, the method may comprise two steps of incubating the tissue with hypotonic treatment. Suitably both steps are for a period of about 21 hours. Suitably at least one of the steps comprises anionic detergent.

As used herein, the term "hypertonic solution" refers to a solution that has a greater concentration of solutes than the concentration of solutes inside of a cell.

Merely by way of example, a hypertonic solution may be selected from, for example, tris/sodium chloride solution (0.6% tris/8-9% sodium chloride solution), sugar solutions, salt solutions, 5% dextrose (sugar) and 0.45% sodium chloride, 5% dextrose and 0.9% sodium chloride, and 10% dextrose in water. In one embodiment, the hypertonic solution is a 0.6% tris/8-9% sodium chloride solution.

In one embodiment, the hypertonic solution is tris/sodium chloride solution (0.6% tris/8.2% sodium chloride solution.

Suitably, the incubation period with the hypertonic solution may be from about 1 hour to about 48 hours, from about 4 hours to 30 hours, from about 8 hours to about 24 hours.

Suitably, the incubation period with the hypertonic solution may be for about 1 hour, about 2 hours, about 3 hours, about 4 hours, about 5 hours, about 6 hours, about 7 hours, about 8 hours, about 9 hours, about 10 hours, about 12 hours, about 14 hours, about 16 hours, about 18 hours, about 20 hours, about 24 hours, about 30 hours, about 32 hours, about 34 hours, about 36 hours, about 38 hours, about 40 hours, about 42 hours, about 44 hours, about 46 hours, about 48 hours, or more.

It will be appreciated that the incubation period with a hypertonic solution may depend upon the type of starting tissue used and/or hypertonic solution. Merely by way of example, porcine dermis may be incubated with a hypertonic solution for about 21-24 hours, whereas porcine meniscus may be incubated with a hypertonic solution for about 8 hours, whereas porcine tendon may be incubated with a hypertonic solution for about 18 hours. The skilled person is able to determine a suitable incubation time by routine experimentation with the tissue in question.

In one embodiment, the incubation period with hypertonic tris/sodium chloride solution (0.6% tris/8.2% sodium chloride solution) is 21-24 hours.

In one embodiment, the incubation period with hypertonic tris/sodium chloride solution (0.6% tris/9% sodium chloride solution) is 8 hours. In one embodiment, the method comprises two incubation periods with hypertonic tris/sodium chloride solution (0.6% tris/9% sodium chloride solution), each for 8 hours.

In one embodiment, the incubation period with hypertonic tris/sodium chloride solution (0.6% tris/8.2% sodium chloride solution) is about 18 hours.

Suitably, the method comprises incubating the tissue at least once with a hypertonic solution. Suitably, the method comprises incubating the tissue one time, two times, three times or more with the hypertonic solution. More suitably, the method may comprise incubating the tissue one time or two times with a hypertonic solution. In one embodiment the method comprises incubating the tissue once with a hypertonic solution.

In an embodiment where the tissue is incubated one time with a hypertonic solution, the step of incubating the tissue with a hypertonic solution may be carried out after incubation with an anionic detergent (with or without a hypotonic solution).

In an embodiment where the tissue is incubated two times with a hypertonic solution, the step of incubating the tissue with a hypertonic solution may be carried out once before incubation with an anionic detergent (with or without a hypotonic solution) and once after incubation with an anionic detergent (with or without a hypotonic solution).

In one embodiment, the method comprises:
- incubating a starting tissue with a delipidating agent;
- incubating the tissue with an anionic detergent and a first hypotonic solution;
- incubating the tissue with a hypertonic solution;
- incubating the tissue with a second hypotonic solution; and
- incubating the tissue with a DNA removal agent, thereby obtaining a decellularized tissue scaffold.

In one embodiment, the method comprises:
- incubating a starting tissue with a delipidating agent;
- incubating the tissue with an anionic detergent and a first hypotonic solution for 24 hours, wherein the first hypotonic solution comprises 0.1% tris/0.1% EDTA solution;
- incubating the tissue with a hypertonic solution for 24 hours, wherein the hypertonic solution comprises 0.6% tris/8-9% sodium chloride solution;
- incubating the tissue with a second hypotonic solution for 24 hours, wherein the second hypotonic solution comprises 0.1% tris/0.1% EDTA solution; and
- incubating the tissue with a DNA removal agent, thereby obtaining a decellularized tissue scaffold.

In one embodiment, the method comprises:
- incubating a starting tissue with a delipidating agent;
- incubating the tissue with a first hypertonic solution;
- incubating the tissue with an anionic detergent and a hypotonic solution;
- incubating the tissue with a second hypertonic solution; and
- incubating the tissue with a DNA removal agent, thereby obtaining a decellularized tissue scaffold.

In one embodiment, the method comprises:
- incubating a starting tissue with a delipidating agent;
- incubating the tissue with a first hypertonic solution for up to 8 hours, wherein the first hypertonic solution comprises 0.6% tris/9% sodium chloride solution;
- incubating the tissue with an anionic detergent and a hypotonic solution for 4 blocks of 20 hours, wherein the hypotonic solution comprises 0.1% tris/0.1% EDTA solution;
- incubating the tissue with a second hypertonic solution for up to 8 hours, wherein the second hypertonic solution comprises 0.6% tris/8-9% sodium chloride solution; and
- incubating the tissue with a DNA removal agent, thereby obtaining a decellularized tissue scaffold.

In one embodiment, the method comprises:
- incubating a starting tissue with a delipidating agent;
- incubating the tissue with a first hypotonic solution;
- subjecting the tissue to one or more freeze/thaw cycles;
- incubating the tissue with a first hypertonic solution;
- incubating the tissue with an anionic detergent and a second hypotonic solution;
- incubating the tissue with a second hypertonic solution; and
- incubating the tissue with a DNA removal agent, thereby obtaining a decellularized tissue scaffold.

In one embodiment, the method comprises:
- incubating a starting tissue with a delipidating agent;
- incubating the tissue with a first hypotonic solution wherein the first hypotonic solution comprises 0.1% tris/0.1% EDTA solution;
- subjecting the tissue to three freeze/thaw cycles;
- incubating the tissue with a first hypertonic solution for up to 8 hours, wherein the first hypertonic solution comprises 0.6% tris/8-9% sodium chloride solution;
- incubating the tissue with an anionic detergent and a second hypotonic solution for 4 blocks of 20 hours, wherein the second hypotonic solution comprises 0.1% tris/0.1 % EDTA solution;
- incubating the tissue with a second hypertonic solution for up to 8 hours, wherein the second hypertonic solution comprises 0.6% tris/8-9% sodium chloride solution; and
- incubating the tissue with a DNA removal agent, thereby obtaining a decellularized tissue scaffold.

### Protease Enzyme Inhibitor

Suitably the method of the invention does not comprise a protease enzyme inhibitor, especially not an animal-derived protease enzyme inhibitor. Therefore, suitably, the process does not comprise a step of incubating the tissue with a protease enzyme inhibitor.

In one embodiment, the method comprises the steps of:
- incubating a starting tissue with a delipidating agent;
- incubating the tissue with an anionic detergent;
- incubating the tissue at least once with a hypotonic solution and at least once with a hypertonic solution, and/or subjecting the tissue to at least one freeze/thaw cycle; and
- incubating the tissue with a DNA removal agent, thereby obtaining a decellularized tissue scaffold,
wherein the method does not comprise an animal-derived protease inhibitor.

However, whilst not necessarily required for the method of the invention, the method may comprise a step of incubating the tissue with a non-animal derived protease enzyme inhibitor. The term "protease enzyme inhibitor" as used herein refers to an agent that inhibits the proteolytic activity of the protease enzyme. The term "non-animal derived" means that the protease inhibitor is not an inhibitor obtained from an animal source, and does not occur naturally in animals.

Suitably, the non-animal derived protease enzyme inhibitor may be a metalloprotease inhibitor. Suitably selected from any chelating agent. Suitably a chelating agent acts as a protease inhibitor and prevents breakdown of the tissue. Suitably the non-animal derived protease inhibitor is selected from the group consisting of EDTA, NTA, ATMP, EDTMP, and HEDP, for example. In one embodiment, the non-animal derived protease inhibitor is EDTA.

Suitably, the non-animal derived protease enzyme inhibitor is used at a concentration from about 0.01% to about 1%, from about 0.05% to about 0.5%, or from about 0.75% to about 0.25%.

Suitably, the non-animal derived protease enzyme inhibitor is used at a concentration of about 1%, about 0.75%, about 0.5%, about 0.25%, about 0.1%, about 0.05% or about 0.01%. In one embodiment, the non-animal derived protease enzyme inhibitor is used at a concentration of about 0.1%.

In a suitable embodiment, non-animal derived protease enzyme inhibitor is EDTA at a concentration of about 0.1%.

It will be appreciated that the step of incubating the tissue with a non-animal derived protease enzyme inhibitor may be carried out before the step of incubating the tissue with a DNA removal agent, or after the step of incubating the tissue with a DNA removal agent.

Suitably, the step of incubating the tissue with a non-animal derived protease enzyme inhibitor may be combined with the step of incubating the tissue with a hypotonic agent.

Suitably, the step of incubating the tissue with a hypotonic solution may be combined with the step of incubating the tissue with a non-animal derived protease enzyme inhibitor. Suitably, the combined incubation of the tissue in a hypotonic solution and a non-animal derived protease enzyme inhibitor is carried out after incubation with a delipidating agent, anionic detergent (with or without hypotonic agent), and hypertonic solution.

In one embodiment, the method comprises the steps of:
- incubating a starting tissue with a delipidating agent for about 2-3 hours, wherein the delipidating agent is 99% (v/v) acetone;
- incubating the tissue with an anionic detergent, wherein the anionic detergent is 0.01% (w/v) SDS;
- incubating the tissue at least once with a hypotonic buffer and at least once with a hypertonic buffer, and/or subjecting the tissue to at least one freeze/thaw cycle; and
- incubating the tissue with a non-animal derived protease enzyme inhibitor, wherein the protease inhibitor is 0.1% EDTA, and incubating the tissue with a DNA removal agent, thereby obtaining a decellularized tissue scaffold.

Suitably, the non-animal derived protease enzyme inhibitor may be diluted to the desired concentration in any suitable solution. Merely by way of example, the non-animal derived protease enzyme inhibitor (for example EDTA) may be diluted in phosphate buffered saline, or in a hypotonic buffer solution (0.1% tris).

It will be appreciated that when the non-animal derived protease enzyme inhibitor (for example EDTA) is diluted in a hypotonic solution, the step of incubating the tissue with a hypotonic solution and a non-animal derived protease enzyme inhibitor may be combined into one step. By way of example, in such an embodiment, the method of the invention comprises the steps of:
- incubating a starting tissue with a delipidating agent;
- incubating the tissue with an anionic detergent;
- incubating the tissue with a hypertonic solution;
- incubating the tissue with a hypotonic solution; and
- incubating the tissue with a non-animal derived protease enzyme inhibitor and a second hypotonic solution, wherein the non-animal derived protease enzyme inhibitor is EDTA; and
- incubating the tissue with a DNA removal agent, thereby obtaining a decellularized tissue scaffold.

In an embodiment, where the non-animal derived protease enzyme inhibitor is not diluted in a hypotonic solution, the method may comprise the following steps:
- incubating a starting tissue with a delipidating agent;
- incubating the tissue with a first hypotonic solution;
- subjecting the tissue to at least one freeze/thaw cycle;
- incubating the tissue with a first hypertonic solution;
- incubating the tissue with an anionic detergent;
- incubating the tissue with a second hypertonic solution;
- incubating the tissue with a DNA removal agent; and
- incubating the tissue with a non-animal derived protease enzyme inhibitor, wherein the non-animal derived protease enzyme inhibitor is EDTA; thereby obtaining a decellularized tissue scaffold.

Optionally the anionic detergent may also comprise a hypotonic solution.

Suitably, the incubation period with a non-animal derived protease enzyme inhibitor (such as EDTA) is sufficient to inhibit proteases in the tissue, suitably this may be from about 0.5 hour to 48 hours, or from about 1 hour to about 24 hours.

Suitably, the incubation period with a non-animal derived protease enzyme inhibitor may be about 0.5 hour, about 1 hour, about 2 hours, about 3 hours, about 4 hours, about 5 hours, about 6 hours, about 7 hours, about 8 hours, about 9 hours, about 10 hours, about 12 hours, about 14 hours, about 16 hours, about 20 hours, about 22 hours, about 24 hours, about 28 hours, about 30 hours, about 36 hours, about 42 hours, or 48 hours, or more.

It will be appreciated that the incubation period with a non-animal derived protease enzyme inhibitor may depend upon the type of starting tissue used and/or the inhibitor itself. Merely by way of example, porcine dermis may be incubated about for 24 hours with an inhibitor such as EDTA, whereas porcine meniscus may be incubated for about 3 hours with an inhibitor such as EDTA. The skilled person is able to determine a suitable incubation time by routine experimentation with the tissue in question.

In one embodiment, the incubation period with a non-animal derived protease enzyme inhibitor 0.1% EDTA is 24 hours.

In one embodiment, the incubation period with a non-animal derived protease enzyme inhibitor 0.1% EDTA is 3 hours.

The inventors have surprisingly found that the method of the invention may produce a decellularized tissue scaffold even when no protease enzyme inhibitor is used, suitably even when only non-animal derived protease inhibitors are used . Aprotinin is a protease enzyme inhibitor, specifically a bovine pancreatic trypsin inhibitor. Due to its bovine origin it may be transmit zoonotic diseases. Suitably the method does not use or comprise an animal-derived protease inhibitor. Suitably the method does not use or comprise aprotinin.

Suitably the method of the invention may further comprise one or more rinse steps to help remove proteases released from lysed cells within the tissue.

Suitably therefore a rinse step may take place after incubating the tissue with a hypotonic solution, after incubating the tissue with a hypertonic solution, and/or after subjecting the tissue to a freeze/thaw cycle.

Suitably, the method comprises a rinse step after each step of cell lysis.

Suitably therefore a rinse step takes place after incubating the tissue with a hypotonic solution, and after incubating the tissue with a hypertonic solution, and after subjecting the tissue to a freeze/thaw cycle. Suitably therefore, the method my comprise multiple rinse steps. Suitably each rinse step may comprise one or more rinses of the tissue. Suitably each rinse step comprises at least three rinses of the tissue.

In one embodiment, the method comprises the steps of:
- incubating a starting tissue with a delipidating agent;
- incubating the tissue with an anionic detergent;
- incubating the tissue at least once with a hypotonic solution and at least once with a hypertonic solution, and/or subjecting the tissue to at least one freeze/thaw cycle;
- rinsing the tissue; and
- incubating the tissue with a DNA removal agent, thereby obtaining a decellularized tissue scaffold.

In one embodiment, the method comprises the steps of:
- incubating a starting tissue with a delipidating agent;
- incubating the tissue with an anionic detergent;
- incubating the tissue at with a hypotonic solution;
- rinsing the tissue;
- incubating the tissue at with a hypertonic solution;
- rinsing the tissue; and
- incubating the tissue with a DNA removal agent, thereby obtaining a decellularized tissue scaffold.

In one embodiment, the method comprises the steps of:
- incubating a starting tissue with a delipidating agent;
- incubating the tissue with an anionic detergent;
- incubating the tissue with a hypotonic solution;
- rinsing the tissue;
- incubating the tissue at with a hypertonic solution;
- rinsing the tissue;
- subjecting the tissue to at least one freeze/thaw cycle;
- rinsing the tissue; and
- incubating the tissue with a DNA removal agent, thereby obtaining a decellularized tissue scaffold.

Suitably rinsing the tissue comprises rinsing the tissue with a rinse solution. Suitably the rinse solution is an isotonic solution. Suitably the rinse solution is a saline solution.

Suitably in each embodiment comprising such one or more rinse steps, the method does not comprise a protease enzyme inhibitor.

In some embodiments, the method may comprise a step of incubating the tissue with a protease or proteinase enzyme. Suitably an animal derived protease or proteinase enzyme. In one embodiment, with a trypsin enzyme, suitably a porcine derived trypsin enzyme. In one embodiment, incubating the tissue with protease or proteinase enzyme is for a period of about 72 hours.

### DNA Removal Agent

The method of the invention comprises the step of incubating the tissue with a DNA removal agent. The term "DNA removal agent" as used herein refers to an agent that denatures and/or cleaves DNA.

Suitably the DNA removal agent may be selected from the group consisting of an endonuclease enzyme, acid, or alkali, for example.

Suitably, the endonuclease enzyme may be a human endonuclease. Suitably the endonuclease enzyme may be selected from the group consisting of DNase Type I, DNase Type II, DNA Type III, RNase or any combination of two or more thereof. Alternatively, the endonuclease enzyme may be a bacterially derived endonuclease, or a combination of human and bacterially derived endonuclease in any combination of two or more thereof. Suitably, the endonuclease may be a DNase I. Suitably, the endonuclease enzyme may be a bacterially derived endonuclease, such as a genetically engineered Seratia marcescens endonuclease which may be selected from any commercially available endonuclease such as Benzonase ^{®}.

Suitably, the endonuclease enzyme may be at a concentration range from about 0.5 to 50 U/ml. In one embodiment, the endonuclease enzyme is at a concentration of about 5 U/ml.

Suitably the endonuclease may be present in a reaction buffer. Suitably the reaction buffer may comprise Tris and magnesium chloride hexahydrate. Suitably the reaction buffer may comprise 0.6% Tris and 0.2% magnesium chloride hexahydrate.

In one embodiment, the method comprises the steps of:
- incubating a starting tissue with a delipidating agent for about 2-3 hours, wherein the delipidating agent is 99% (v/v) acetone;
- incubating the tissue with an anionic detergent, wherein the anionic detergent is 0.01% (w/v) SDS;
- incubating the tissue at least once with a hypotonic buffer and at least once with a hypertonic buffer, and/or subjecting the tissue to at least one freeze/thaw cycle; and
- incubating the tissue with a DNA removal agent wherein the DNA removal agent is 5 U/ml of DNase I, thereby obtaining a decellularized tissue scaffold.

Suitably, the incubation period with a DNA removal agent is for a sufficient time to remove DNA to a level of less than 50ng/mg. Suitably this may be from about 2 to about 24 hours, suitably from about 2 to about 12 hours. Suitably, the incubation period with a DNA removal agent may be for about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, or more.

It will be appreciated that the incubation period with a DNA removal agent may depend upon the type of starting tissue used and/or the agent itself. Merely by way of example, porcine dermis may be incubated for about 3 hours with a DNA removal agent such as an endonuclease, whereas porcine meniscus may be incubated for about 22 hours with a DNA removal agent such as an endonuclease, whereas porcine tendon may be incubated for about 6 hours. The DNA may be sufficiently removed from the tissue if the DNA amount is less than about 50 ng/mg. The skilled person will appreciate that removal of DNA from the tissue may be optimised through routine experiments involving different concentrations and/or incubation times with a DNA removal agent.

In one embodiment, the incubation period with the DNA removal agent 5U/ml of endonuclease is 3 hours.

In one embodiment, the incubation period with the DNA removal agent 5U/ml of endonuclease is 22 hours, comprised of two 2 hour blocks of time and one 18 hour block of time.

In one embodiment, the incubation period with the DNA removal agent 5U/ml of endonuclease is 6 hours, comprised of three 2 hour blocks of time.

In one embodiment, the DNA removal agent is Benzonase ^{®}.

The step of incubating the tissue with a DNA removal agent may be performed before or after the step of incubating the tissue with a non-animal derived protease enzyme inhibitor, if present.

### Antimicrobial Agent

The method of the invention may further comprise the step of incubating the tissue in an antimicrobial agent. This step may be particularly beneficial in the context of a method for producing a decellularized tissue scaffold from a non-human starting tissue as such tissues are associated with the risk of transmitting zoonotic disease.

The term "antimicrobial agent" as used herein, refers to any natural, synthetic, or semisynthetic compound that has antibacterial, antifungal, antiviral, and/or antiparasitic activity. In the context of the present disclosure, such activity may include decreasing the number of living bacteria, fungi, viruses, and/or parasites in the starting tissue and/or tissue scaffold, and/or decreasing the chance of contamination and subsequent infection of recipient of the tissue scaffold upon use *in vivo.* Decreasing the number of living microorganisms may be achieved by limiting, preventing and/or inhibiting the growth of the microorganisms, and/or killing of the microorganisms.

Suitably, the antimicrobial agent may be an oxidizing agent. The term "oxidizing agent" as used herein refers to an agent that is capable of oxidizing a cellular membrane of a microorganism, resulting in microorganism cell lysis and death.

Suitably, the oxidizing agent may be peracetic acid (PAA). Suitably, the PAA may be at a concentration from about 500 to about 10,000 ppm. Suitably the PAA may be at a concentration of about 500ppm to 1500ppm. In one embodiment, the PAA is at a concentration of 1000ppm, otherwise written as 0.1%.

Suitably, the oxidizing agent (for example PAA) may have a pH from about 6 to about 8. More suitably, the oxidizing agent (for example PAA) may have a pH of about 7.

Suitably the oxidizing agent may be present in a buffer, suitably for example PBS buffer.

It will be appreciated that the antimicrobial agent may comprise a single antimicrobial agent or two or more antimicrobial agents. For example, the antimicrobial agent may comprise two antimicrobial agents.

In an embodiment where the antimicrobial agent comprises two or more antimicrobial agents, at least one of the agents may be a mild oxidizing agent (for example PAA). Suitably, in an embodiment where the antimicrobial agent comprises two or more antimicrobial agents, at least one of the agents may be a mild oxidizing agent (for example PAA) and at least one of the agents may be an antibiotic.

Suitably, the antibiotic may be selected form the group consisting of penicillin streptomycin, streptomycin, ampicillin, actinomycin D, carbenicillin, cefotaxime, fosmidomycin, gentamicin, kanamycin, neomycin and polymyxin B.

In the method of the invention, the tissue may be incubated with an antimicrobial agent (such as a mild oxidizing agent, for example PAA) for a sufficient time until the tissue is essentially free from living microorganisms. Suitably, the incubation may be from about 15 minutes to about 24 hours, suitably from about 30 minutes to about 12 hours, suitably from about 1 hour to about 6 hours, suitably from about 2 to about 6 hours.

More suitably, the incubation with an antimicrobial agent (such as an oxidizing agent, for example PAA) may be for about 1 hour. In this context, "essentially free from" means that the tissue is at least 80%, at least 85%, at least 90%, at least 95%, or at least 99%, or more devoid of microorganisms which exist in the tissue prior to incubation with an antimicrobial agent. The skilled person is able to determine the appropriate incubation time to remove microorganisms from the tissue, for example by routine experimentation.

In one embodiment, the incubation with an antimicrobial agent of 0.1% PAA is 1 hour or about 3 hours.

The step of incubating with an antimicrobial agent may be performed at least once, twice, three times, or more. Suitably the step of incubating with an antimicrobial agent may be performed once or twice. Merely by way of example, the incubation with an antimicrobial agent (such as an oxidizing agent, for example PAA) may be performed prior to incubation with a delipidating agent and/or after incubation with a protease enzyme inhibitor or DNA removal agent. Suitably incubation with PAA may be directly before packaging of the decellularized tissue scaffold. Additionally, further incubation with an antimicrobial agent may be performed after a step of subjecting the tissue to at least one freeze/thaw cycle.

In one embodiment, the method comprises the steps of:
- optionally incubating a starting tissue with an antimicrobial agent;
- incubating the tissue with a delipidating agent;
- incubating the tissue with an anionic detergent;
- incubating the tissue at least once with a hypotonic buffer and at least once with a hypertonic buffer, and/or subjecting the tissue to at least one freeze/thaw cycle;
- incubating the tissue with a DNA removal agent; and
- incubating the tissue with an antimicrobial agent, thereby obtaining a decellularized tissue scaffold.

In another embodiment, the method comprises the steps of:
- incubating a starting tissue with a delipidating agent for about 2-3 hours, wherein the delipidating agent is 99% (v/v) acetone;
- incubating the tissue with an anionic detergent, wherein the anionic detergent is 0.01% (w/v) SDS;
- incubating the tissue at least once with a hypotonic buffer and at least once with a hypertonic buffer, and/or subjecting the tissue to at least one freeze/thaw cycle;
- incubating the tissue with a DNA removal agent wherein the DNA removal agent is 5 U/ml of DNase I; and
- incubating the tissue with an antimicrobial agent, wherein the antimicrobial agent is 0.1% PAA, thereby obtaining a decellularized tissue scaffold.

In one embodiment, the method may comprise two steps of incubating with an antimicrobial agent. Suitably wherein the antimicrobial agent is 0.1% PAA. Suitably the first step comprises incubating with the antimicrobial agent for a period of about 1 hour. Suitably the second step comprises incubating with the antimicrobial agent for a period of about 3 hours.

### Ionizing Radiation

Animal tissues must be generally exposed to a disinfection/sterilization process that has been proven to reduce zoonosis by 6-log. However, reduction of this magnitude is not typically achieved by traditional sterilisation methods e.g. gamma irradiation at a dose of 25 kGy. The inventors have surprisingly found that such a level of sterilisation maybe achieved using by ionizing radiation. Accordingly, the method of the invention may further comprise the step of subjecting the decellularized biological scaffold to ionizing radiation. Suitably the ionizing radiation may be from about 15 kGy to about 50kGy. Suitably, the ionizing radiation may be at about 18kGy to 25 kGy, suitably the ionizing radiation may be between 25kGy to 27.5kGy

Suitably, the ionizing radiation may be performed before or after the tissue has been packaged. Suitably, the ionizing radiation may be performed after the tissue has been packaged.

Suitably, the ionizing radiation may be performed with or without dry ice.

In one embodiment, the method comprises the steps of:
- incubating a starting tissue with a delipidating agent;
- incubating the tissue with an anionic detergent;
- incubating the tissue at least once with a hypotonic buffer and at least once with a hypertonic buffer, and/or subjecting the tissue to at least one freeze/thaw cycle;
- incubating the tissue with a DNA removal agent;
- incubating the tissue with an antimicrobial agent to thereby obtaining a decellularized tissue scaffold;
- optionally packaging the scaffold; and
- subjecting the scaffold to ionizing radiation.

In another embodiment, the method comprises the steps of:
- incubating a starting tissue with a delipidating agent for about 2-3 hours, wherein the delipidating agent is 99% (v/v) acetone;
- incubating the tissue with an anionic detergent, wherein the anionic detergent is 0.01% (w/v) SDS;
- incubating the tissue at least once with a hypotonic buffer and at least once with a hypertonic buffer, and/or subjecting the tissue to at least one freeze/thaw cycle;
- incubating the tissue with a DNA removal agent wherein the DNA removal agent is 5 U/ml of DNase I;
- incubating the tissue with an antimicrobial agent, wherein the antimicrobial agent is 0.1% PAA, thereby obtaining a decellularized tissue scaffold;
- optionally packaging the scaffold; and
- subjecting the scaffold to ionising radiation of about 18kGy to 25 kGy, suitably 25kGy to 27.5kGy.

The skilled person will appreciate that between each of the incubation steps of the method of the invention the tissue may be rinsed or washed at least once. Suitably, the tissue may be rinsed or washed at least two times or three times between each of the incubation steps. Merely by way of example rinsing or washing may be with saline. Alternatively, rinsing or washing may be with a buffer, for example a wash buffer or well-known buffer such as PBS. Suitably the saline may comprise 0.9% sodium chloride solution. Suitably the wash buffer may comprise 0.1% EDTA and 0.1% phosphate buffered saline solution. Suitably the PBS may comprise 0.1% phosphate buffered saline solution.

### Particular Embodiments of the Invention

In one embodiment, the method may be adapted for a porcine tendon tissue scaffold. Suitably wherein the starting tissue is porcine tendon.

In one embodiment, the method of the invention comprises:
(a) incubating a starting tissue with a delipidating agent;
(b) incubating the tissue with a hypotonic solution;
(c) subjecting the tissue to at least one freeze/thaw cycle;
(d) incubating the tissue with an antimicrobial agent;
(e) incubating the tissue with a hypotonic solution;
(f) incubating the tissue with an anionic detergent;
(g) incubating the tissue with a hypotonic solution;
(h) incubating the tissue with an anionic detergent;
(i) incubating the tissue with a DNA removal agent;
(j) incubating the tissue with a hypertonic solution;
(k) incubating the tissue with an antimicrobial agent.

In one embodiment, the delipidating agent is acetone, suitably 99% (v/v) acetone. In one embodiment, step (a) comprises incubating a starting tissue with a delipidating agent for about 2 hours. In one embodiment, step (a) comprises incubating a starting tissue with a delipidating agent for three rounds, suitably each round comprising about 40 minutes.

In one embodiment, the hypotonic solution is a hypotonic tris buffer comprising 0.1% Tris and 0.1% EDTA. In one embodiment, step (b) comprises incubating the tissue with a hypotonic solution for about 1-4 hours. In one embodiment, step (e) comprises incubating the tissue with a hypotonic solution for about 24 hours. In one embodiment, step (g) comprises incubating the tissue with a hypotonic solution for about 24 hours.

In one embodiment, the method comprises two freeze/thaw cycles. Suitably each cycle is performed in a hypotonic solution.

In one embodiment, the method may comprise a step of storage of the tissue. Suitably the tissue may be stored after being frozen. Suitably this may take place at the end of the method, or part way through the method, suitably part way through step (c). Suitably the storage step may be for a period of up to 12 months.

In one embodiment, the antimicrobial agent is PAA, suitably 0.1% PAA. In one embodiment, step (d) comprises incubating the tissue with an antimicrobial agent for about 1 hour. In one embodiment step (k) comprises incubating the tissue with an antimicrobial agent for about 3 hours.

In one embodiment, the anionic detergent is SDS, suitably 0.1% (w/v) SDS. In one embodiment, the SDS is present in a hypotonic solution, suitably in a Tris buffer. In one embodiment step (f) comprises incubating the tissue with an anionic detergent for about 24 hours. In one embodiment step (h) comprises incubating the tissue with an anionic detergent for about 24 hours.

In one embodiment, the DNA removal agent is 5 U/ml of Benzonase^{®} in buffer, the buffer may comprise 0.6% Tris and 0.2% magnesium chloride hexahydrate. In one embodiment step (i) comprises incubating the tissue with a DNA removal agent for about 6 hours. In one embodiment step (i) comprises incubating the tissue with a DNA removal agent for three rounds, suitably each round comprising about 2 hours.

In one embodiment, the hypertonic solution is 0.6% Tris and 8.2% sodium chloride. In one embodiment, step (j) comprises incubating the tissue with a hypertonic solution for about 18 hours.

In one embodiment, one or more washing steps are present in the method as described above. In one embodiment, a washing step may be present between steps (a) and (b), between steps (h) and (i), between steps (i) and (j), between steps (j) and (k), and/or after step (k). In one embodiment the washing steps between step (a) and (b), between steps (h) and (i), and after step (k) are with saline. In one embodiment the washing step between steps (i) and (j) is with a wash buffer. In one embodiment the washing step between steps (j) and (k) is with PBS.

Suitably most of the steps of the method are performed at around 37°C. Suitably however step (a) and step (b) are conducted at between 18-30°C.

Suitably the method further comprises a step of terminal sterilisation as described elsewhere herein.

In another embodiment, the method may be adapted for a porcine dermis tissue scaffold. Suitably wherein the starting tissue is porcine dermis.

In one embodiment, the method of the invention comprises:
(a) incubating a starting tissue with an antimicrobial agent;
(b) incubating the tissue with a delipidating agent;
(c) incubating the tissue with a proteinase;
(d) incubating the tissue with a delipidating agent;
(e) incubating the tissue with an anionic detergent;
(f) incubating the tissue with a hypertonic solution;
(g) incubating the tissue with a hypotonic solution;
(h) incubating the tissue with a DNA removal agent;
(i) incubating the tissue with an antimicrobial agent.

In one embodiment, the antimicrobial agent is PAA, suitably 0.1% PAA. In one embodiment, step (a) comprises incubating the tissue with an antimicrobial agent for about 1 hour. In one embodiment step (i) comprises incubating the tissue with an antimicrobial agent for about 3 hours.

In one embodiment, the delipidating agent is acetone, suitably 99% (v/v) acetone. In one embodiment, step (b) comprises incubating a starting tissue with a delipidating agent for about 33 hours. In one embodiment, step (b) comprises incubating a starting tissue with a delipidating agent for three rounds, suitably a first and second round of about 1 hour, and a third round of about 31 hours. In one embodiment, step (d) comprises incubating a starting tissue with a delipidating agent for about 3 hours. In one embodiment, step (d) comprises incubating a starting tissue with a delipidating agent for three rounds, suitably each round comprising about 1 hour.

In one embodiment the proteinase is a natural proteinase. In one embodiment the proteinase is trypsin, suitably 0.2% porcine derived trypsin in buffer, the buffer may comprise 0.6% Tris and 0.2% magnesium chloride hexahydrate. In one embodiment, step (c) comprises incubating the tissue with a proteinase for about 72 hours.

In one embodiment, the anionic detergent is SDS, suitably 0.01% (w/v) SDS. In one embodiment, the SDS is present in a hypotonic solution, suitably in a Tris buffer. In one embodiment step (e) comprises incubating the tissue with an anionic detergent for about 21 hours.

In one embodiment, the hypertonic solution is 0.6% Tris and 8.2% sodium chloride. In one embodiment, step (f) comprises incubating the tissue with a hypertonic solution for about 21 hours.

In one embodiment, the hypotonic solution is 0.1% Tris and 0.1% EDTA. In one embodiment, step (g) comprises incubating the tissue with a hypotonic solution for about 21 hours.

In one embodiment, the method may further comprise a step of dehairing the tissue. Suitably this step may take place between steps (c) and (d). Suitably the dehairing may take up to 8 hours. Suitably at least one washing step is present before and after the dehairing step.

In one embodiment, the method may further comprise a holding step, suitably wherein the tissue is held in saline. Suitably the holding step may take place prior to final step (i). Suitably the holding step may be up to 72 hours.

In one embodiment, the DNA removal agent is 5 U/ml of Benzonase^{®} in buffer, the buffer may comprise 0.6% Tris and 0.2% magnesium chloride hexahydrate. In one embodiment step (h) comprises incubating the tissue with a DNA removal agent for about 3 hours.

In one embodiment, one or more washing steps are present in the method as described above. In one embodiment, a washing step may be present between steps (a) and (b), between steps (b) and (c), between steps (c) and (d), between steps (d) and (e), between steps (g) and (h), between steps (h) and (i), and/or after step (i). In one embodiment the washing steps are with saline.

Suitably most of the steps of the method are performed at around 14°C. Suitably however step (a) and step (i) are conducted at about 27°C. Suitably however step (b) and step (d) are conducted at about 21.5°C.

Suitably the method further comprises a step of terminal sterilisation as described elsewhere herein.

### Decellularized Tissue Scaffold

The present invention provides a decellularized tissue scaffold produced by a method as described herein.

The present inventors have surprisingly found that the method of the invention removes anionic detergent (such as SDS) residues from the tissue. Without wishing to be bound to this hypothesis, the inventors believe that the removal of anionic detergent residues is possible due to optimisation of process conditions used in other steps of the method, in particular the process conditions used in the incubations with a hypertonic and hypotonic solution and/or the freeze/thaw cycle.

Accordingly, the decellularized tissue scaffold of the invention is substantially free from anionic detergent (such as SDS) residues. By 'substantially free' it is meant that the tissue scaffold is at least 90%, at least 95%, at least 96%, at least 97%, at least 98%, at least 99%, or up to 100% free of processing residues from anionic detergents. Suitably, by virtue of the method described herein, the tissue scaffold of the invention comprises less anionic detergent residue than those scaffolds produced in the art.

Accordingly, the decellularized tissue scaffold of the invention is substantially free from galactose-alpha-1,3-galactose (also known as alpha-gal) protein. By 'substantially free' it is meant that the tissue scaffold may comprise 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, 1% or less of alpha-gal as compared to the amount of alpha-gal naturally occurring in the starting tissue from which the scaffold is produced. Suitably, by virtue of the method described herein, the tissue scaffold of the invention comprises less alpha-gal protein residue than those scaffolds produced in the art.

Suitably, the tissue scaffold is a complex tissue scaffold. In one embodiment, the complex tissue scaffold is a meniscus tissue scaffold.

### EXAMPLES

### Example 1: Porcine Dermis

Pre-treatment: Porcine dermis was dissected and sliced to 1-2mm in thickness.

The tissue was washed in 0.1% peracetic acid (PAA) for 1 hour followed by being washed with saline.

The tissue was the washed with 99% (v/v) acetone three times for 1 hour, followed by three washes with saline to remove the acetone.

The tissue was then incubated in 0.2% trypsin for 72 hours to allow manual dehairing, followed by a further three washes with saline and three washes with acetone.

De-cellularisation: The tissue was washed in 0.01% SDS in hypotonic tris buffer for up to 24 hours followed by a wash with hypertonic solution of 0.6% tris/9% sodium chloride solution for 24 hours and a further wash of hypotonic buffer (0.1% tris/0.1% EDTA) for 24 hours.

DNA was removed with a wash of 5000 U/I endonuclease for 3 hours followed by three washes in saline.

The tissue was cut to size prior to viral inactivation in 0.1% PAA, final packing and terminal sterilisation at 25 kGy.

To demonstrate effective decellularisation, the tissue was tested for DNA residue using the Qiagen DNAeasy Blood and Tissue Kit and NanoQuant system and also stained with haematoxylin and eosin (Figure 1). An average DNA content of 16ng/mg was demonstrated. <50ng/mg is recognised as the standard for decellularization.

### Example 2: Porcine Meniscus

Pre-treatment: Porcine meniscus was dissected and sliced to 7-8mm in thickness.

The tissue was washed three times in 99% (v/v) acetone for 40 minutes each, followed by three washes in saline to remove the acetone.

The tissue was then washed in a hypotonic tris buffer for 40 minutes and subjected to three freeze/thaw cycles. The tissue was frozen at -80°C.

De-cellularisation: The tissue was thawed and washed in 0.1% peracetic acid (PAA) for 1 hour. The tissue was then washed in a hypertonic solution of 0.6% tris/9% sodium chloride solution for up to 8 hours followed by incubation in 0.01% SDS in hypotonic tris buffer for up to 20 hours 4 times followed by a wash in hypertonic solution of 0.6% tris/9% sodium chloride solution for up to 8 hours. The tissue was then washed in saline for 40 minutes x 3 times. DNA was removed with a wash of 5000 U/I endonuclease for 2 hours x2, followed by an endonuclease incubation of 18 hours followed by three saline washes.

Processing residues were removed from the tissue by incubation of the tissue in a solution of 0.01% EDTA/0.1% phosphate buffered saline for up to 3 hours followed by seven washes in saline and three washes of phosphate buffered saline for 40 minutes.

The tissue was washed in 0.1% PAA to ensure viral inactivation followed by three washes in saline and one final saline wash for 90 hours prior to final packing and terminal sterilisation at 25 kGy.

To demonstrate effective decellularisation, the tissue was tested for DNA residue using the Qiagen DNAeasy Blood and Tissue Kit and NanoQuant system and also stained with heamatoxylin and eosin. An average DNA content of 14ng/mg was demonstrated, whereas <50ng/mg is recognised as the standard for decellularization.

Figure 1 shows tissue that decellularization has been achieved whilst maintaining the histoarchitecture of the material. Figure 1 A shows a decellularized meniscus scaffold. Figure 1 B shows a decellularized dermis scaffold. In both cases the histoarchitecture of the tissue has been retained. Figure 1 C is another view of the scaffold of figure 1B.

### Experimental data

### 1. Alpha-gal characterization of Porcine Dermis

α-gal was quantified using the MyBioSource α-gal ELISA kit (MBS262885). Tissue decellularized by the method of example 1 was first homogenized using a high shear mixer with extraction and treatment using the standard α-gal ELISA kit method. Following extraction, α-gal was quantified by measuring absorbance of the sample at 450nm.

**Table 1**

| α-gal in untreated dermis | α-gal in d-cell processed dermis | % reduction α-gal |
|---|---|---|
| 16.07 ng | 2.98 ng | 81.5% |

As can be seen from the results in Table 1, the method of the invention significantly reduced the amount of alpha-gal in the tissue scaffold.

### 2. Comparison of the method of the invention with the method described in EP1392372B1

The cytotoxicity of a tissue decellularized in accordance with EP1392372B1, and a tissue decellularized in accordance with example 2 herein, were evaluated using ISO 10993-5 (extract method).

**Table 2**

| Process | DNA Content | SDS Residue % w/w | Cytotoxicity |
|---|---|---|---|
| V1 (Prior art) | 19.5 ng/mg | 0.75 | Grade 4 (Fail) |
| V2 (From example 2) | 8.4 ng/mg | 0.002 | Grade 0 (Pass) |

| | | | |
|---|---|---|---|
| Note: Cytotoxicity is graded 0-4, 4 being more cytotoxic. Products up to Grade 2 are acceptable as medical devices but Grade 3 & 4 are unacceptable. | | | |

As can be seen from the data in Table 2, the method of the invention as carried out in example 2 (V2) is better at removing the DNA content and better at removing the cytotoxic SDS residues than the prior art method.
**The present application and invention further includes the subject matter of the following numbered clauses:**
1. A method for producing a decellularized tissue scaffold, the method comprising the steps of:
   - incubating a starting tissue with a delipidating agent;
   - incubating the tissue with an anionic detergent;
   - incubating the tissue at least once with a hypotonic solution and at least once with a hypertonic solution, and/or subjecting the tissue to at least one freeze/thaw cycle; and
   - incubating the tissue with a DNA removal agent, thereby obtaining a decellularized tissue scaffold.
2. The method according to Paragraph 1, wherein the starting tissue is selected from the group consisting of: skin, meniscus, tendon, ligament, cartilage, muscle, blood vessel, and an organ.
3. The method according to Paragraph 1 or Paragraph 2, wherein the delipidating agent is a polar solvent.
4. The method according to Paragraph 3, wherein the polar solvent is acetone.
5. The method according to any one of the preceding Paragraphs, wherein the concentration of the delipidating agent is from about 80% to about 100% (v/v).
6. The method according to any one of the preceding Paragraphs, wherein the anionic detergent is sodium dodecyl sulphate (SDS) or sodium deoxycholate.
7. The method according to any one of the preceding Paragraphs, wherein the concentration of the anionic detergent is equal to or less than 0.2% (w/v).
8. The method according to any preceding Paragraph, wherein the hypotonic solution comprises 0.1% tris/0.1% EDTA.
9. The method according to any preceding Paragraph, wherein the tissue is incubated with a hypotonic solution for between 30 minutes to 100 hours, preferably for between 10 hours to 100 hours, preferably for between 20 hours to 80 hours.
10. The method according to any preceding Paragraph, wherein the hypertonic solution comprises 0.6% tris/8-9% sodium chloride solution.
11. The method according to any preceding Paragraph, wherein the tissue is incubated with a hypertonic solution for between 1 hour to 48 hours, preferably for between 4 hours to 30 hours, preferably for between 8 hours to 24 hours.
12. The method according to any one of the preceding Paragraphs, wherein the method does not comprise an animal-derived protease inhibitor.
13. The method according to any preceding Paragraph, wherein the method further comprises one or more steps of rinsing the tissue.
14. The method according to Paragraph 13, wherein the method comprises a step of rinsing the tissue after each step of: incubating with a hypotonic solution, incubating with a hypertonic solution, and subjecting the tissue to at least one freeze/thaw cycle.
15. The method according to any preceding Paragraph, wherein the endonuclease enzyme is human or bacterial, preferably wherein the endonuclease is selected from the group consisting of DNase Type I, DNase Type II, DNA Type III, RNase, or any combination of two or more thereof.
16. The method according to Paragraph 15, wherein the endonuclease enzyme is at a concentration from about 0.5 to about 50 U/ml, preferably 5 U/ml.
17. The method according to any one of the preceding Paragraphs, wherein the method further comprises the step of incubating the tissue with an antimicrobial agent.
18. The method according to Paragraph 17, wherein the antimicrobial agent is an oxidizing agent, preferably wherein the oxidizing agent is a peracetic acid.
19. The method according to any one of Paragraphs 17 to 18, wherein the antimicrobial agent is at a concentration from about 0.05% (w/v) to 1% (w/v).
20. The method according to any one of the preceding Paragraphs, further comprising the step of subjecting the tissue to ionizing radiation, preferably wherein the ionizing radiation is at a dose of from about 15 to about 50 kGy, preferably about 25 kGy.
21. A decellularized tissue scaffold produced by a method according to any one of Paragraphs 1 to 20.
22. The decellularized tissue scaffold according to Paragraph 21, wherein the decellularized tissue scaffold is substantially free from anionic detergent residues.
23. The decellularized tissue scaffold according to Paragraph 22, wherein the anionic detergent residues are SDS residues.
24. The decellularized tissue scaffold according to any of Paragraphs 21-23, wherein the tissue scaffold is a meniscus tissue scaffold, a dermis tissue scaffold, or a tendon tissue scaffold.
25. The decellularized tissue scaffold according to Paragraph 24, wherein the meniscus tissue scaffold is a porcine meniscus tissue scaffold, a porcine dermis tissue scaffold or a porcine tendon tissue scaffold.

## Claims

1. A decellularized tissue scaffold which is at least 80% devoid of lipids and/or lipid-like substances compared to the starting tissue obtained from a human or animal subject used to make the decellularized tissue scaffold, wherein the starting tissue is selected from the group consisting of: skin, meniscus, tendon, ligament, cartilage, muscle, and blood vessel.

2. The decellularized tissue scaffold according to claim 1, wherein the decellularized tissue scaffold is substantially free from anionic detergent residues.

3. The decellularized tissue scaffold according to claim 2, wherein the anionic detergent residues are SDS residues.

4. The decellularized tissue scaffold according to any of claims 1-3, wherein the decellularized tissue scaffold is at least 90% free from anionic detergent residues.

5. The decellularized tissue scaffold according to any of claims 1-4, wherein the decellularized tissue scaffold is substantially free from protease enzyme inhibitors.

6. The decellularized tissue scaffold according to claim 5, wherein the protease enzyme inhibitors are animal-derived protease enzyme inhibitors.

7. The decellularized tissue scaffold according to any of claims 1-6, wherein the decellularized tissue scaffold is substantially free from galactose-alpha-1,3-galactose (alpha-gal) protein.

8. The decellularized tissue scaffold according to any of claims 1-7, wherein the decellularized tissue scaffold comprises 20% or less of alpha-gal as compared to the amount of alpha-gal naturally occurring in the starting tissue from which the scaffold is produced.

9. The decellularized tissue scaffold according to any of claims 1-8, wherein the tissue scaffold is a meniscus tissue scaffold, a dermis tissue scaffold, or a tendon tissue scaffold.

10. The decellularized tissue scaffold according to claim 9, wherein the meniscus tissue scaffold is a porcine meniscus tissue scaffold, a porcine dermis tissue scaffold or a porcine tendon tissue scaffold.

11. The decellularized tissue scaffold of any of claims 1-10, wherein the decellularized tissue is at least 80% devoid of cellular components.

12. The decellularized tissue scaffold of any of claims 1-11, wherein the decellularized tissue comprises DNA in an amount of less than about 50ng/mg dry weight of the scaffold.

13. The decellularized tissue scaffold of any of claims 1-12, wherein the decellularized tissue scaffold is produced by a method comprising the steps of:
• incubating a starting tissue with a delipidating agent wherein the delipidating agent is a polar solvent;
• incubating the tissue with an anionic detergent;
• incubating the tissue at least once with a hypotonic solution and at least once with a hypertonic solution, and/or subjecting the tissue to at least one freeze/thaw cycle; and
• incubating the tissue with a DNA removal agent, thereby obtaining a decellularized tissue scaffold; wherein the starting tissue is selected from the group consisting of: skin, meniscus, tendon, ligament, cartilage, muscle, and blood vessel.
